(19) Europäisches Patentamt
European Patent Office
Office européen des brevets

(11) **EP 2 465 416 B1**

(12) **FASCICULE DE BREVET EUROPEEN**

(45) Date de publication et mention
de la délivrance du brevet:
**20.11.2013 Bulletin 2013/47**

(51) Int Cl.:
*A61B 5/00* *(2006.01)*     *G01N 21/47* *(2006.01)*
*G01N 21/64* *(2006.01)*     *G01N 33/58* *(2006.01)*

(21) Numéro de dépôt: **11193342.0**

(22) Date de dépôt: **13.12.2011**

(54) **Procédé de localisation d'un marqueur optique dans un milieu diffusant**

Lokalisierungsverfahren eines optischen Markers in einem Diffusionsmedium

Method for locating an optical marker in a diffusing medium

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **15.12.2010 FR 1060567**

(43) Date de publication de la demande:
**20.06.2012 Bulletin 2012/25**

(73) Titulaire: **Commissariat à l'Énergie Atomique
et aux Énergies Alternatives
75015 Paris (FR)**

(72) Inventeurs:
• **Laidevant, Aurélie
74150 RUMILLY (FR)**
• **Dinten, Jean-Marc
69008 LYON (FR)**
• **Montcuquet, Anne-Sophie
38000 GRENOBLE (FR)**

(74) Mandataire: **Ilgart, Jean-Christophe et al
Brevalex
95, rue d'Amsterdam
75378 Paris Cedex 8 (FR)**

(56) Documents cités:
**EP-A1- 1 884 765         JP-A- 2008 215 881
US-A1- 2008 103 390      US-B2- 7 672 834**

• **HERVE L ET AL: "Localization of fluorescence
marked prostate tumor with time-resolved diffuse
optical tomography", PROCEEDINGS OF SPIE,
SPIE, US, vol. 7557, 23 février 2010 (2010-02-23),
pages 75570F-1, XP002617075, ISSN: 0277-786X,
DOI: 10.1117/12.840861**
• **MONTCUQUET A -S ET AL: "Non-negative matrix
factorization: a blind sources separation method
to unmix fluorescence spectra", 2009 FIRST
WORKSHOP ON HYPERSPECTRAL IMAGE AND
SIGNAL PROCESSING: EVOLUTION IN REMOTE
SENSING (WHISPERS) IEEE PISCATAWAY, NJ,
USA, 2009, page 4 PP., XP002651714, ISBN:
978-1-4244-4686-5**
• **FALCONET J ET AL: "Estimation of optical
properties of turbid media: experimental
comparison of spatially and temporally resolved
reflectance methods", APPLIED OPTICS
OPTICAL SOCIETY OF AMERICA USA, vol. 47, no.
11, 10 avril 2008 (2008-04-10), pages 1734-1739,
XP002651715, ISSN: 0003-6935**

**Description**

**DOMAINE TECHNIQUE ET ART ANTERIEUR**

L'invention concerne le domaine de l'imagerie optique, par exemple de fluorescence, dans les milieux diffusants et notamment les tissus biologiques. Elle met en oeuvre des méthodes optiques résolues en temps.

Elle trouve une application au domaine médical, en particulier à la tomographie optique diffusive de fluorescence pour la détermination de la distribution de particules fluorescentes, par exemple des marqueurs.

L'imagerie optique diffuse, et notamment l'imagerie de fluorescence, offre la perspective de systèmes de diagnostic non invasifs grâce à l'utilisation de rayonnements non-ionisants, faciles d'utilisation et peu coûteux.

Dans les applications mettant en oeuvre l'imagerie de fluorescence, des marqueurs fluorescents sont injectés au sujet et se fixent sur certaines molécules spécifiques, par exemple des tumeurs cancéreuses. La zone d'intérêt est éclairée à la longueur d'onde d'excitation optimale du fluorophore (substance chimique d'une molécule capable d'émettre de la lumière de fluorescence après excitation) et le signal fluorescent est détecté.

On cherche ensuite, à partir de ce signal de fluorescence à obtenir des informations de localisation ou de concentration sur les marqueurs.

L'imagerie optique de fluorescence (avec injection de fluorophore spécifique) est limitée à ce jour à des applications « petit animal » en raison du manque de marqueurs adaptés et injectables à l'humain, et du problème d'auto-fluorescence des tissus qui se pose pour la détection en profondeur.

En effet, pour appliquer cette méthode au diagnostic de cancers chez l'homme, il est primordial que le signal spécifique situé plus profondément sous la peau que chez le petit animal puisse être détecté.

Mais le signal spécifique à détecter faiblit avec la profondeur, principalement à cause de l'absorption et de la diffusion des tissus, et se confronte à un signal parasite qui perturbe la détection. Ce signal, appelé « auto-fluorescence », décrit la fluorescence de tissus auxquels aucune substance chimique ou fluorophore spécifique n'a été injecté : il s'agit de la fluorescence naturelle du tissu. Lorsque les marqueurs sont situés en profondeur (quelques cm), leur signal spécifique s'atténue. Alors, l'autofluorescence peut devenir d'amplitude non négligeable car elle est émise par tout le volume des tissus biologiques.

De plus, le filtrage de la lumière d'excitation n'est jamais parfait et un peu de lumière d'excitation peut passer à travers le filtre de fluorescence censé la supprimer et venir s'ajouter au signal mesuré. D'autres éléments du système de mesure (filtres par exemple) peuvent également avoir une faible fluorescence propre.

Ces deux phénomènes, autofluorescence propre des tissus et signal parasite dû au système, sont des phénomènes qui viennent interférer et fausser la mesure de fluorescence.

L'autofluorescence est un phénomène connu, mais est à ce jour rarement perçue comme un signal parasite. En cancérologie notamment, l'autofluorescence est utilisée pour distinguer des tissus cancéreux de tissus sains. Il ne s'agit alors pas d'injecter de marqueur spécifique, mais simplement d'observer l'autofluorescence de zones spécifiques et de comparer différentes zones d'un même individu.

Par opposition, la spectroscopie optique de fluorescence utilise généralement des longueurs d'onde d'excitation en rouge ou proche infra rouge, qui assurent une absorption moindre du signal dans les tissus, et permettent ainsi une meilleure pénétration des tissus. L'autofluorescence des tissus est alors beaucoup plus faible et devient un signal à supprimer plutôt qu'à utiliser.

Dans la suite on pourra regrouper les deux effets que sont l'autofluorescence propre des tissus et la production de signaux parasites dus au système sous le terme de signaux parasites.

On connait différentes méthodes de soustraction d'images pour limiter l'effet de l'autofluorescence dans le cas d'une illumination du milieu par une source continue.

Selon une première de ces méthodes on réalise une soustraction simple du signal moyen d'une région de l'image qui ne contient pas de marqueurs fluorescents. Une limitation de ces techniques est la nécessité de trouver une zone de référence sans fluorescence représentative et de reproduire des conditions d'acquisition similaires.

Selon un autre type de méthode on effectue une soustraction en utilisant des filtres « de fond » décalés vers le bleu : cette technique est mise en place dans les systèmes IVIS de Caliper Life Science. Une image est prise avec un filtre décalé vers le bleu dont le rôle est d'exciter l'autofluorescence, mais pas les fluorophores. Cette image de fond est ensuite soustraite - à un facteur d'échelle près - à l'image régulière avec le filtre d'excitation. Il n'est pas certain que cette fluorescence produite par un décalage vers le bleu soit représentative de l'autofluorescence à la longueur d'onde d'observation.

On trouve aussi des méthodes à base de techniques spectrales, mais elles nécessitent évidemment de réaliser des acquisitions résolues en longueur d'onde.

Ainsi une technique multispectrale avec des excitations à différentes longueurs d'onde a été décrite dans le document US 2005065440.

D'autres travaux, voir notamment ceux de D.Wood et al. « Refining epifluorescence imaging and analysis with automated multiple-band flat-field correction", paru dans Nature Methods Application Notes (2008), ou S. Psycharakis et al., Autofluorescence removal from fluorescence tomography data using multispectral imaging (Proceedings Paper SPIE 66260I-66260I-7 (2007)) utilisent également une méthode multispectrale basée sur des modèles a priori de spectres. Certains de ces travaux ont présenté des résultats en tomographie du petit animal.

Enfin, il existe des méthodes de décomposition spectrale (mesure à une seule longueur d'onde) pour distinguer l'autofluorescence de la fluorescence. Un spectromètre permet de mesurer le spectre d'émission en chaque pixel de l'image. La décomposition sépare les deux spectres et permet d'obtenir deux images, une d'autofluorescence, une de fluorescence.

Certaines de ces techniques effectuent une séparation de spectres par analyse en Composantes Principales (ACP), d'autres par Factorisation en Matrices Non Négatives. Là encore, la méthode ne s'applique qu'à des illuminations continues c'est-à-dire non pulsées, la factorisation en matrices non négatives permettant de reconstruire le spectre en longueur d'onde de différentes composantes constituant un signal de fluorescence détecté. Par spectre en longueur d'onde, on entend une distribution en énergie. (US 20090245611).

D'autres techniques mettent en oeuvre une illumination du milieu par une lumière pulsée, également appelée fluorescence résolue en temps. Par lumière pulsée, on entend une impulsion lumineuse dont la durée est de quelques fs à quelques ns. Dans ce cas, la détection du signal émis par le tissu s'effectue généralement entre deux impulsions consécutives de la source lumineuse.

Ainsi, dans le document A.T. Kumar et al. « Feasibility of in vivo imaging of fluorescent proteins using lifetime contrast", Optics Letters 34, 2066-2068 (2009), on distingue la fluorescence de l'autofluorescence (AF) sur un petit animal en modélisant la distribution de l'AF et le déclin de fluorescence du fluorophore. Mais cette technique met en oeuvre une connaissance a priori des profils de déclin pour faire un ajustement sur les mesures temporelles. Cette technique permet de réaliser seulement une image en deux dimensions, mais ne permettent pas d'accéder à une information sur la localisation en profondeur des espèces d'intérêt.

Enfin, dans la technique décrite dans l'article de A. Laidevant et al. « Analytical method for localizing a fluorescent inclusion in a turbid medium », Applied Optics, 46, 2131-2137 (2007), une mesure spécifique du signal parasite est effectuée sur le milieu en retirant l'inclusion fluorescente. Cette mesure est ensuite soustraite au signal de fluorescence brute. Cette technique est évidemment coûteuse en temps (elle nécessite de réaliser une mesure sans inclusion fluorescente puis une mesure avec inclusion fluorescente) et est délicate à mettre en oeuvre.

HERVE L ET AL: "Localization of fluorescence marked prostate tumor with time-resolved diffuse optical tomography", PROCEEDINGS OF THE SPIE vol. 7557, 23 février 2010 (2010-02-23) décrit une méthode de fluorescence résolue en temps.

A, MONTCUQUET A -S ET AL: "Non-negative matrix factorization: a blind sources separation method to unmix fluorescence spectra", 2009 FIRST WORKSHOP ON HYPERSPECTRAL IMAGE AND SIGNAL PROCESSING: EVOLUTION IN REMOTE SENSING (WHISPERS) IEEE PISCATAWAY, NJ, USA, 2009, JP 2008/215881 et US 2008/103390 décrivent des méthodes de factorisation en matrices non-négatives.

D'une manière générale, il se pose donc le problème de trouver un nouveau procédé, ne nécessitant pas la mise en oeuvre d'une technique spectrale, pouvant être réalisée rapidement (en vue d'application in vivo) et permettant de différencier, les différentes contributions de sources lumineuses, et notamment les contributions de sources fluorescentes et de sources parasites, en faisant appel à une technique de fluorescence résolue en temps.

Il se pose également le problème de trouver un nouveau dispositif, permettant de mettre en oeuvre un tel procédé.

## EXPOSÉ DE L'INVENTION

L'invention concerne d'abord un procédé de traitement de donnée relatives à au moins un marqueur optique dans un milieu diffusant, dans lequel :

a) on réalise au moins une acquisition d'un signal optique résolu en temps, ce signal résultant d'une excitation du milieu à l'aide d'une source de rayonnement en impulsions, chaque acquisition comportant d'une part une composante d'intérêt, due à au moins un marqueur, et d'autre part une composante parasite,
b) on constitue une matrice X à partir de chaque acquisition réalisée à l'étape a).

On peut ensuite traiter les données de ladite matrice X par factorisation de cette matrice en un produit de deux matrices non négatives A et S.

Il est ensuite possible d'extraire, ou de déterminer ou de calculer ou d'identifier, la contribution d'une desdites composantes à au moins une desdites acquisitions, à partir des termes des matrices A et S.

Le signal optique est caractéristique du milieu et d'un ou plusieurs desdits marqueurs. Il résulte d'une interaction du milieu avec un rayonnement d'excitation, en impulsions, chaque acquisition comportant d'une part une ou plusieurs composantes d'intérêt, due à un ou plusieurs marqueurs, d'autre part une composante parasite, due à une partie du milieu autre que les marqueurs.

Selon un mode de réalisation, un tel procédé peut en outre comporter une étape de calcul ou de détermination ou d'identification ou de localisation de la position d'au moins un marqueur optique dans le milieu diffusant et/ou une représentation graphique de la répartition d'au moins un tel marqueur dans le milieu diffusant, à partir d'au moins une desdites composantes temporelles acquises.

Selon ce mode de réalisation, l'invention permet donc une identification de la contribution au signal détecté, mais aussi une identification (même approchée) de la position, d'un ou de plusieurs marqueurs optiques dans le milieu diffusant étudié, en trois dimensions, sans avoir recours à une décomposition spectrale du rayonnement de fluorescence, et sans nécessiter de mesures temporelles censées ne représenter que le signal parasite (sans le marqueur).

Un marqueur peut être une molécule présentant les propriétés optiques souhaitées.

Selon un mode de réalisation, au moins un des marqueurs optiques est un marqueur fluorescent.

Dans ce cas, au cours de l'étape a), on détecte un signal de fluorescence, dont une longueur d'onde correspond à au moins une longueur d'onde d'au moins un marqueur fluorescent, le milieu diffusant étant alors excité par une source de rayonnement en impulsions, la source émettant à une longueur d'onde d'excitation de la fluorescence du marqueur.

La composante d'intérêt, due à au moins un des marqueurs fluorescents, est alors au moins une composante de fluorescence (mais il peut y en avoir plusieurs).

La composante parasite peut comprendre une composante d'autofluorescence du milieu, ainsi qu'une composante de diffusion de la lumière d'excitation.

On peut donc ensuite extraire ou calculer ou déterminer ou identifier une contribution de fluorescence due à au moins l'un des marqueurs fluorescents à partir des termes des matrices A et S.

On peut ensuite déterminer ou calculer la position de l'un au moins des marqueurs fluorescents, dans le milieu diffusant, et/ou une représentation graphique d'une répartition d'un ou de plusieurs marqueurs fluorescents, à partir de la contribution de fluorescence obtenue lors de l'étape d).

Selon un autre mode de réalisation, au moins un des marqueurs optiques présente des propriétés d'absorption et/ou de diffusion différentes de celles du milieu dans lequel il est placé. Au moins une des composantes d'intérêt comporte alors un rayonnement absorbé et/ou diffusé.

Dans ce cas, le rayonnement détecté est un rayonnement de diffusion émis par le milieu, en réponse à une excitation lumineuse impulsionnelle. Dans ce cas le rayonnement diffusé par le marqueur présente des caractéristiques temporelles distinctes de celles du rayonnement diffusé par le milieu.

On peut ensuite déterminer ou calculer la position de l'un au moins des marqueurs, dans le milieu environnant, et/ou une représentation graphique d'une répartition d'un ou de plusieurs marqueurs, à partir des composantes d'intérêt.

Un procédé selon l'invention peut comporter, avant formation dudit tableau multidimensionnel, une étape de correction des données de signal optique de chaque acquisition par recentrage, ou correction, des données temporelles en fonction, ou autour, d'un temps moyen du signal optique, par exemple par décalage temporel des données, ce décalage ayant une valeur égale à un temps de réponse impulsionnel.

Dans l'un ou l'autre des procédés ci-dessus, la première matrice A non négative du produit AS peut être une matrice dont les éléments $a_{x,p}$ sont des coefficients de pondération, $a_{x,p}$ étant la contribution, dans une acquisition indicée par l'indice x, d'une composante d'intérêt, par exemple de fluorescence, ou d'une composante de diffusion et/ou d'autofluorescence, ces composantes étant désignées par l'indice p.

La seconde matrice non négative S peut comporter le profil temporel d'intérêt, par exemple de fluorescence, de chaque composante.

Chaque ligne p de cette matrice peut représenter un profil temporel de signal caractéristique de la composante p (des valeurs numérisées de ce profil temporel). Le nombre de lignes du tableau S et le nombre de colonnes du tableau A correspondant alors au nombre de composantes à séparer.

Le tableau X est formé en réalisant des acquisitions successives, une acquisition pouvant par exemple correspondre à une position donnée de la source et à une position donnée du détecteur, ou à la position d'une source donnée et à la position d'un détecteur donné (ici et dans le reste du texte, la position d'une source (respectivement : d'un détecteur) peut-être la position de l'extrémité distale d'une fibre qui transmet un rayonnement depuis ladite source vers le milieu étudié (ou depuis ce milieu vers le détecteur).

Ainsi, la matrice X comprend les données temporelles mesurées, ces dernières étant également appelées acquisitions, indicées par l'indice x et notées $X_x$. Chaque acquisition correspond à un histogramme temporel du signal détecté par le détecteur.

Les matrices X, A et S sont généralement de dimension 2.

Lors de l'étape de traitement du tableau X comprenant les données temporelles mesurées, ces dernières étant également appelées acquisitions, on peut déterminer A et S par minimisation d'une fonction de coût F, cette fonction pouvant être :

- le carré de la distance euclidienne entre la matrice X et le produit matriciel A.S : on a alors $F = \|X - AS\|^2$
- ou bien encore la fonction :

$$F = \|X - AS\|^2 + \alpha\|S - S_0\|^2$$

où $S_0$ est la matrice initiale de S et $\alpha$ un coefficient réel dit de régularisation.

La matrice X, rassemblant une ou plusieurs acquisitions, est traitée de préférence selon un procédé itératif. Par exemple, on réalise k itérations, les tableaux $A_i$ et $S_i$, obtenus lors de l'itération d'ordre i étant réactualisés à chaque itération. Le nombre d'itération peut être déterminé en fonction de l'évolution de la fonction de coût F entre des itérations successives, ou en fonction de la valeur de cette fonction de coût, en la comparant alors à un critère d'arrêt prédéterminé. Autrement dit, dans ce dernier cas, le processus itératif s'arrête lorsque la fonction de coût atteint ledit critère d'arrêt. Ce nombre d'itérations peut également être déterminé empiriquement, en fonction de l'expérience de l'utilisateur.

L'excitation du milieu peut être réalisée par une source d'excitation laser, qui peut être éventuellement focalisée à l'interface entre le milieu diffusant et le milieu extérieur. La lumière d'excitation va ensuite pénétrer dans le milieu diffusant, et y exciter des marqueurs ou des sources dans ce milieu, par exemple à 3 cm ou 5 cm en profondeur, c'est-à-dire à distance de l'interface, dans le milieu diffusant. Le rayonnement de fluorescence provient donc d'une zone en profondeur, par exemple comprise entre l'interface et environ 3 cm ou 5 cm à distance de l'interface, ou comprise entre 1 cm à distance de l'interface et 5 cm à distance de l'interface. L'excitation peut avoir lieu dans l'infrarouge ou le proche infra rouge ou le rouge, par exemple à au moins une longueur d'onde comprise environ entre 600 et 900 nm. Selon un mode de réalisation, la fluorescence d'un marqueur fluorescent peut quant à elle être détectée à des longueurs d'onde supérieures à la longueur d'onde d'excitation du milieu.

L'invention concerne également un dispositif de localisation d'un marqueur optique dans un milieu diffusant, ou de traitement de données relatives à un tel marqueur, comportant :

a) au moins une source de rayonnement en impulsions pour produire un faisceau d'excitation du milieu diffusant,
b) des moyens pour réaliser au moins une acquisition d'un signal de diffusion optique résolu en temps d'au moins un marqueur optique dudit milieu, l'acquisition comportant d'une part une ou plusieurs composantes d'intérêt, et d'autre part une ou plusieurs composantes parasite,
c) des moyens pour constituer une matrice X à partir de chaque acquisition,
d) des moyens pour traiter les données de ladite matrice X par factorisation en deux matrices non négatives A et S,
e) des moyens pour calculer ou déterminer ou identifier ou extraire la contribution d'une desdites composantes à au moins une desdites acquisitions à partir des termes des matrices A et S.

Selon un mode de réalisation, un tel dispositif comprend en outre des moyens pour déterminer ou calculer ou identifier et/ou pour visualiser la répartition des intensités du ou des marqueurs optiques à partir d'au moins une contribution d'une desdites composantes.

Le marqueur optique peut être un marqueur fluorescent. Dans ce cas, la composante d'intérêt associée à ce marqueur est une composante de fluorescence. Aussi, la source d'excitation produit des impulsions à la longueur d'onde d'excitation du marqueur fluorescent, et les moyens de détection sont adaptés pour collecter le rayonnement de fluorescence du marqueur fluorescent.

Selon ce mode, la source d'excitation permet d'éclairer le milieu, et de produire une zone, dite zone d'excitation dans ce dernier. En effet la lumière d'excitation pénètre dans le milieu, y diffuse, et excite les sources de fluorescence, marqueurs et tissus autofluorescents. Comme expliqué ci-dessus, les sources de fluorescence peuvent être localisées en profondeur, à distance sous l'interface.

Un dispositif selon l'invention peut comporter en outre des moyens pour modifier la position de la source d'excitation et/ou des moyens de détection. Une acquisition du signal optique de diffusion résolu en temps, par exemple un signal de fluorescence, peut être réalisée pour chaque position de la source d'excitation et des moyens de détection.

Ou bien un dispositif selon l'invention peut comporter plusieurs fibres optiques qui permettent d'amener le rayonnement de la source d'excitation en différents points du milieu et/ou diverses fibres optiques qui permettent de capter, en différents points du milieu ou en différents points de sa limite, un rayonnement optique, par exemple un rayonnement de fluorescence, qui provient de ce milieu, et de l'amener à un ou plusieurs détecteurs.

Les moyens pour traiter la matrice d'acquisition X par factorisation en deux matrices non négatifs A et S, mettent en oeuvre un procédé selon l'invention, comme déjà décrit ci-dessus.

## BRÈVE DESCRIPTION DES DESSINS

- La figure 1 représente un dispositif pour mettre en oeuvre l'invention,
- les figures 2A et 2B représentent un autre dispositif pour mettre en oeuvre l'invention,
- les figures 3A-3B des acquisitions, d'une part de la fluorescence brute mesurée et, d'autre part, du signal parasite mesuré, pour deux couples source - détecteur,
- la figure 4 est un exemple de superposition d'un ensemble d'acquisition de fluorescence brute, pour 6 sources et 4 détecteurs,
- la figure 5 représente schématiquement la décomposition,
- la figure 6 illustre comment est constituée une acquisition de fluorescence,
- la figure 7 représente un milieu et des zones à propriétés optiques différentes du milieu environnant,
- les figures 8A-8B et 9A-9B représentent des comparaisons entre des composantes de fluorescence mesurées et des composantes de fluorescence calculées, ainsi qu'entre des composantes parasites mesurées et calculés par la mise en oeuvre d'un procédé selon l'invention,
- les figures 10A, 10B et 11A, 11B représentent des images de reconstruction, d'une part sans correction de la composante parasite (figure 9 A) puis avec correction de la composante parasite (figure 9 B) et, d'autre part, avec correction expérimentale de la composante parasite (figure 10A) et avec correction selon la présente invention (figure 10B).

## EXPOSÉ DÉTAILLÉ DE MODES DE REALISATIONS DE L'INVENTION

Dans cet exposé, on va tout d'abord considérer le cas où le (ou les) marqueurs optiques sont des marqueurs fluorescents.

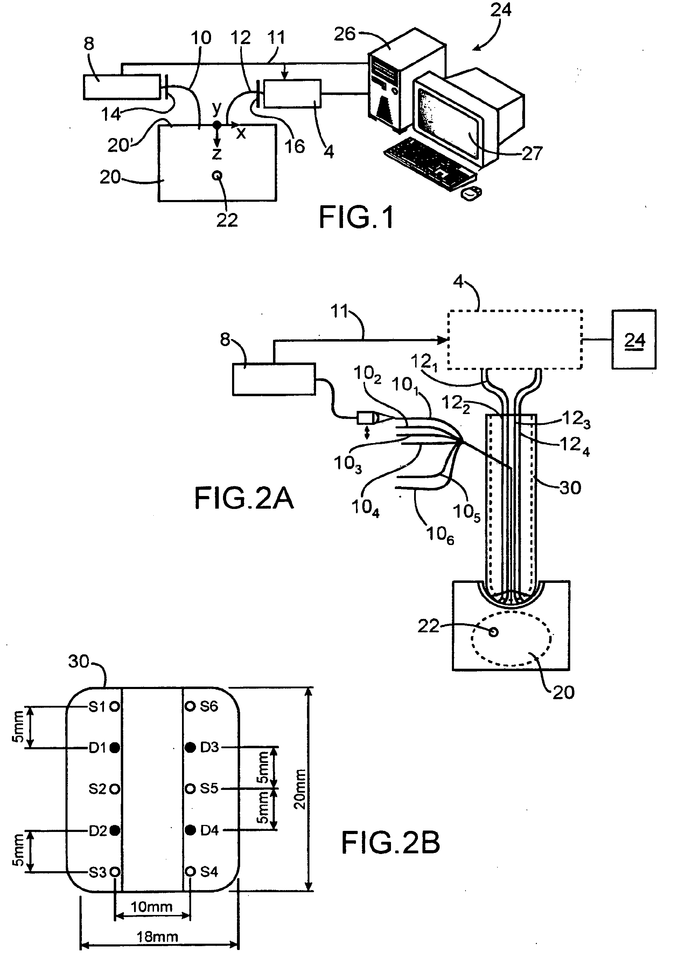

La figure 1 est un exemple de système expérimental permettant de mettre en oeuvre l'invention.

L'illumination d'un milieu 20 à examiner est réalisée à l'aide d'une source 8 de rayonnement qui produit des impulsions de rayonnement, par exemple un rayonnement dans l'infra rouge ou même le proche infra rouge. Cette source de rayonnement est impulsionnelle, chaque impulsion de rayonnement pouvant avoir une largeur à mi-hauteur comprise entre, par exemple, quelques nanosecondes, par exemple 1 ns ou 10 ns, et quelques femtosecondes, par exemple 10 fs.

Il peut par exemple s'agir d'un laser fonctionnant de manière impulsionnelle. Le faisceau de cette source de rayonnement peut être dirigé vers le milieu, par exemple avec une fibre optique 10.

En variante, plusieurs fibres peuvent être utilisées pour envoyer des impulsions à divers endroits dans le milieu, définissant alors autant de sources de lumière, chaque extrémité de fibre pouvant être assimilée à une source de lumière s. Des moyens de commutation, par exemple une platine de translation, permettent alors la sélection d'une fibre parmi les différentes fibres.

Une platine permet de positionner la source de rayonnement devant telle ou telle fibre.

Le rayonnement d'excitation passe de préférence par un filtre interférentiel 14 pour éliminer une grande partie de la lumière à une longueur d'onde supérieure à la longueur d'onde d'excitation.

La lumière d'excitation diffuse dans le milieu diffusant 20 et va y exciter une ou plusieurs espèces fluorescentes.

En réponse, le milieu émet un rayonnement comprenant :

- une composante de fluorescence, due à la présence du ou des marqueurs fluorescents 22, cette composante étant parfois appelée fluorescence spécifique car elle provient des marqueurs fluorescents. C'est cette composante que l'on cherche généralement à déterminer, de façon à localiser la position des marqueurs fluorescents dans le milieu,
- une composante parasite. Cette dernière peut notamment comporter une composante d'autofluorescence du milieu, et/ou une composante de diffusion du rayonnement d'excitation dans le milieu et/ou une ou plusieurs composantes parasites dues par exemple à la fluorescence propre de certains éléments matériels du système, par exemple des composants optiques.

Une ou plusieurs fibres 12 collectent la lumière en provenance du milieu 20 étudié, dite lumière émise. Un filtre interférentiel 16 et/ou un filtre coloré peuvent être placés devant le détecteur 4 pour limiter la détection de lumière en dehors du spectre de fluorescence des fluorophores 22 disposés dans le milieu 20 et optimiser l'élimination de la lumière d'excitation. Le filtre peut par exemple être un filtre passe haut, laissant passer les longueurs d'onde supérieures à 650 nm lorsque la source émet à la longueur d'onde 631 nm.

Le rayonnement émis est donc envoyé vers des moyens de détection 4 et d'acquisition 24. Les moyens de détection 4 comprennent un détecteur de photons, relié à des moyens d'acquisition du signal détecté, ces derniers pouvant comporter des cartes d'acquisition d'un ordinateur.

Ces moyens d'acquisition 24 permettent d'obtenir la distribution temporelle du signal détecté par les moyens de détection 4. Par distribution temporelle, on entend le temps, dit temps d'arrivée, séparant la détection d'un photon d'un temps initial $t_0$ déterminé. Ce temps initial correspond généralement à l'instant $t_{imp}$ où la source émet une impulsion lumineuse, éventuellement avec un léger décalage temporel. Une telle distribution est généralement discrétisée dans le temps et se présente sous la forme d'un histogramme communément dénommé par l'acronyme TPSF (Temporal Point Spread Function). D'une façon générale, les moyens de détection et d'acquisition permettent d'obtenir un signal de fluorescence résolu en temps, ce signal prenant généralement la forme de l'histogramme précédemment décrit.

Eventuellement le rayonnement émis est transmis vers les moyens de détection 4 via une ou plusieurs fibres optiques 12, comme déjà expliqué ci-dessus. Dans ce cas, l'extrémité de chaque fibre optique située du côté du milieu diffusant peut être assimilée à un détecteur.

Les moyens de détection 4 et d'acquisition 24 comportent par exemple un photomultiplicateur couplé à une carte de comptage, cette dernière produisant une distribution temporelle des signaux détectés par le photomultiplicateur.

Les moyens de détection 4 et d'acquisition 24 peuvent également comporter une caméra rapide intensifiée, cette caméra étant activée selon une porte temporelle dont l'ouverture est successivement décalée, ce qui permet de réaliser simultanément un histogramme de type TPSF en différents points de l'espace.

Une ligne de synchronisation 11 permet de déclencher les moyens de détection 4 et/ou d'acquisition 24 à l'aide d'une impulsion engendrée par la source de rayonnement 8.

Ainsi, ces moyens de détection 4 et d'acquisition 24 permettent d'obtenir, un signal de fluorescence résolu en temps, ou histogramme temporel du signal de fluorescence. Lorsque le milieu est excité par une source s et que le signal émis par le milieu est détecté par le détecteur d, cet histogramme est noté $X_{sd}$. Il peut être également être appelé acquisition résolue en temps correspondant au couple source-détecteur. Ainsi, une acquisition résolue en temps peut être référencée par $X_x$ ou $X_{sd}$, l'indice x étant un entier avec $1 \leq x \leq Nx$, l'indice sd représentant un couple source-détecteur.

Ces moyens 24 comportent des moyens pour digitaliser ou numériser les données temporelles de fluorescence. Des moyens 26 de traitement des données vont permettre de mettre en oeuvre un procédé de traitement selon l'invention. Ces moyens électroniques 24 comportent par exemple un ordinateur ou un micro ordinateur ou un microprocesseur programmé pour mémoriser et traiter les données acquises par les moyens 4. Ils peuvent également comporter des moyens de synchronisation permettant de synchroniser l'acquisition du signal détecté par le détecteur 4 et les impulsions de la source impulsionnelle 8. Une unité centrale 26 est programmée pour mettre en oeuvre un procédé de traitement selon l'invention. Des moyens 27 d'affichage ou de visualisation permettent, après traitement, de représenter le positionnement ou la distribution spatiale des fluorophores dans le milieu examiné. Les moyens 4, 24 permettent éventuellement de commander ou de contrôler d'autres parties du dispositif expérimental, par exemple la sélection de la position de la source et/ou du détecteur, par exemple encore via la position d'une platine ou d'un commutateur optique.

Le milieu étudié 20 est un milieu diffusant, par exemple un tissu biologique. Un rayonnement incident, d'excitation, peut pénétrer dans ce type de milieu, la profondeur de pénétration pouvant atteindre quelques cm selon le coefficient d'atténuation de ce milieu, par exemple 3 cm ou 5 cm.

Autrement dit, des fluorophores situés à une distance z de la frontière 20' du milieu comprise entre 0 cm (donc situés très proches de la surface) et, par exemple 3 cm ou 5 cm vont pouvoir être détectés.

Les moyens de détection 4 et d'acquisition 24, déterminent la distribution temporelle $X_{sd}$ d'un rayonnement qui provient de la zone du milieu diffusant excitée par la source lumineuse s, qui traverse le milieu diffusant en direction de la frontière 20' entre le milieu diffusant et le milieu extérieur, puis qui atteint les moyens 4 de détection comprenant le détecteur d. Une analyse spectrale n'est pas nécessaire pour les besoins de la présente invention.

Typiquement, le milieu étudié peut être un milieu vivant. Il peut s'agir par exemple d'une zone du corps humain ou animal. L'enveloppe corporelle constitue l'interface du milieu diffusant avec le milieu extérieur. Une source d'excitation est donc focalisée sur cette interface, ou dans le milieu. Des marqueurs préalablement injectés dans ce milieu diffusant permettent de localiser des zones telles des tumeurs.

Comme déjà expliqué ci-dessus, il y a également excitation d'autres éléments du milieu, créant une composante parasite au signal détecté.

Un autre exemple de dispositif selon l'invention est illustré en figure 2A, dans une application à un milieu qui est en fait un fantôme de prostate 20 contenant des marqueurs fluorescents 22. Un laser 8 fonctionne en impulsions, par exemple à une longueur d'onde proche de 775 nm. Les impulsions envoyées sont très brèves : elles sont par exemple de l'ordre de quelques dizaines de picosecondes (par exemple de largeur temporelle à mi-hauteur comprise entre 10 ps et 50 ps).

La sortie du laser 8 est fibrée et envoyée dans 6 fibres sources $10_1$, $10_2$, $10_3$, $10_4$, $10_5$, $10_6$. Ces fibres sont intégrées dans une coque de sonde endorectale 30 en contact avec le fantôme. Pour chaque fibre source ($10_i$, i=1-6), la lumière se propage dans le milieu 20 jusqu'à la zone fluorescente.

Les marqueurs fluorescents 22 ainsi excités émettent des photons à une longueur d'onde supérieure à celle d'excitation, ici aux environs de 800 nm. Mais il y a dans le signal d'autres contributions parasites, notamment de l'autofluorescence qui provient des tissus biologiques environnant les marqueurs fluorescents. En utilisant un fantôme tel que celui représenté sur la figure 2A, il est possible d'obtenir un signal d'autofluorescence en effectuant la soustraction de deux acquisitions réalisées respectivement avec et sans l'inclusion fluorescente.

Cette sonde contient également 4 fibres de détection $12_j$ (j=1-4) pour collecter le signal de fluorescence. Ces fibres acheminent la lumière détectée vers le détecteur d compris dans les moyens de détection 4, relié à une carte de comptage de photons, faisant partie des moyens d'acquisition 24, qui permet de construire l'histogramme des temps d'arrivée des photons ou distribution temporelle des photons. Les moyens de détection 4, et d'acquisition 24 ont déjà été décrits ci-dessus. Des filtres peuvent aussi être utilisés, comme déjà expliqué ci-dessus.

La figure 2B représente l'extrémité 30' de la sonde 30, avec les extrémités $S_1$ - $S_6$ des fibres d'excitation $10_i$, i=1-6 et celles ($D_1$ - $D_4$) des fibres de détection $12_j$ (j=1-4) .

À titre d'exemple, l'extrémité de la sonde 30 peut avoir une section sensiblement rectangulaire, par exemple de côté 20 mm et 18 mm. Les extrémités des fibres d'excitation et de détection sont réparties selon deux rangées, de manière alternée, sur chaque rangée, entre les fibres d'excitation et les fibres de détection, par exemple selon un pas d'environ 5 mm. On a donc, sur chaque rangée, les extrémités de 5 fibres, dont 3 fibres d'excitation et 2 fibres de détection.

Pour chaque fibre d'excitation $10_i$, i=1-6 on a pu réaliser des mesures par chaque fibre de détection $12_j$ (j=1-4) .

Ainsi, dans l'exemple de 6 fibres d'excitation et de 4 fibres de détection, on a pu réaliser 4 mesures (une pour chaque fibre de détection $12_j$ (j=1-4)) avec une excitation par la fibre $10_i$, i=1-6 (i=1 - 6).

Dans la suite de la description, le terme source désigne à la fois une source non fibrée, ou l'extrémité d'une fibre d'excitation lorsque la source est fibrée. De même, le terme détecteur désigne à la fois un détecteur, un groupe de pixels d'un détecteur pixelisé, ou l'extrémité d'une fibre de détection, optiquement couplée à un détecteur.

On identifie dans la suite (ou sur les figures) par « source n, détecteur p » (ou $S_n$, $d_p$) une acquisition obtenue par excitation via source d'excitation $10_n$ et par détection via le détecteur $12_p$. Aussi, chaque acquisition est notée $X_{s_n d_p}$. Dans la suite de la description, $X_{sndp}$ est un vecteur-ligne, chaque ligne comportant des données représentant l'histogramme temporel de fluorescence, c'est-à-dire une distribution temporelle des photons détectés. $X_{sndp}$ pourrait également être représenté sous la forme d'un vecteur colonne.

Chaque acquisition $X_{s_n d_p}$ est constituée de termes notés $X_{s_n d_p 't}$, $t$ représentant le pas de discrétisation temporel, avec $1 \leq t \leq Nt$. Nt est l'abscisse maximum d'un histogramme, correspondant généralement à un temps d'arrivée de quelques ns à 10 ns environ.

Chaque acquisition pourrait être également indicée par un indice x, et serait alors notée $X_x$, avec $1 \leq x \leq Nx$, Nx étant le nombre total d'acquisitions assemblées dans la matrice d'acquisitions X.

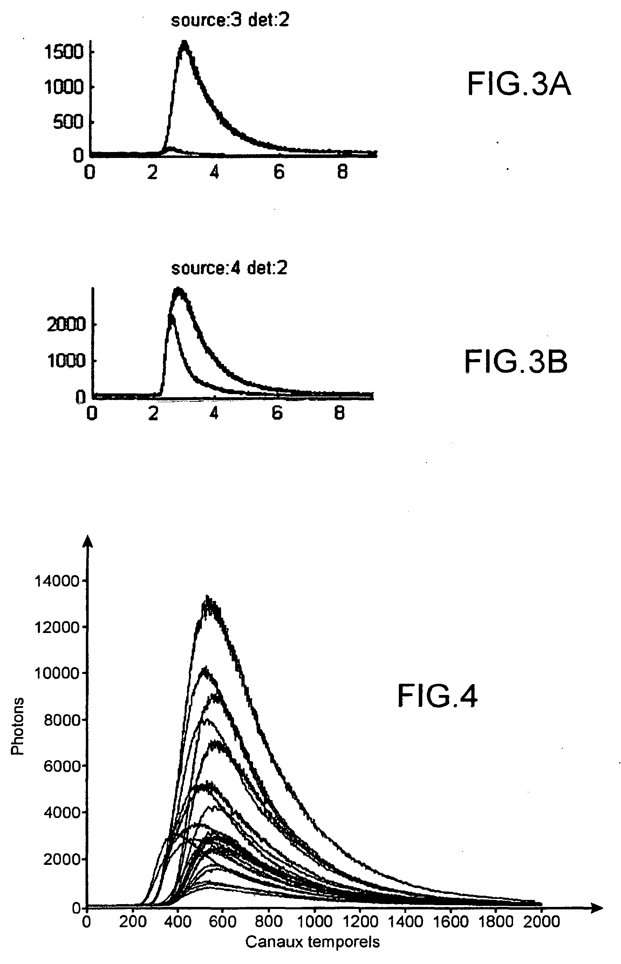

On a donc obtenu, dans le cas des figures 2A et 2B, 4 x 6 = 24 acquisitions.

Pour chaque configuration, correspondant à un couple source détecteur, on mesure :

- une acquisition dite de fluorescence brute, provenant du milieu avec l'inclusion fluorescente ; Le terme brut désigne le fait que le signal détecté comprend une composante de fluorescence, ou composante d'intérêt, et une composante parasite. Ce signal résolu en temps correspond à l'acquisition $X_{sd}$,
- une acquisition constituée uniquement de ladite composante parasite, ce signal étant mesuré sans l'inclusion fluorescente ; notons que, en général, la détermination d'une telle composante n'est pas possible, car il n'est pas possible de retirer les marqueurs fluorescents du milieu,
- une acquisition $X_{diff}$ constituée de la diffusion du signal d'excitation dans le milieu (à la longueur d'onde d'excitation, sans filtre de fluorescence).

Quelques exemples d'acquisitions obtenues avec ce dispositif sont illustrés en figures 3A - 3B :

- pour la source 2 et le détecteur 2 (figure 3A),
- pour la source 4 et le détecteur 2 (figure 3B).

Chacune de ces figures montre simultanément le signal (résolu en temps) de fluorescence brut et le signal (résolu en temps) parasite. Ce dernier est parfois faible par rapport au signal de fluorescence brute (cas de la figure 3A) mais parfois important (cas de la figure 3B). On voit qu'il y a donc bien ici une perturbation des mesures de signal de fluorescence par les signaux parasites d'autofluorescence et/ou de diffusion.

Pour traiter ce problème, on peut réaliser d'abord un recalage temporel des données d'acquisition temporelles des signaux de fluorescence brute $X_{sd}$, par rapport à un temps de réponse impulsionnel.

Cette étape de correction est optionnelle, mais sera de préférence réalisée. Les inventeurs ont en effet constaté qu'un tel recalage temporel de chaque acquisition $X_{sd}$, réalisé préalablement à la décomposition de la matrice X, permettait d'améliorer les résultats de la factorisation de cette matrice.

Un objectif de ce recalage temporel peut être de s'affranchir des variations des fonctions de réponse impulsionnelle du dispositif, ou de l'ensemble constitué par le dispositif et le milieu, entre les différents couples sources-détecteurs.

En effet, dans une configuration telle que décrite dans la figure 2A, les sources et les détecteurs sont fibrés. Selon la longueur d'une fibre d'excitation 10i séparant le Laser 8 de l'extrémité de la sonde 30, selon la longueur d'une fibre d'émission 12i s'étendant entre l'extrémité de la sonde 30 des moyens de détection 4, et selon les positions des extrémités desdites fibres d'excitation 10i et d'émission 12i sur la sonde 30, une même impulsion du Laser ne sera pas détectée au même moment selon qu'on utilise un couple fibre d'émission - fibre de détection (ou couple source-détecteur) ou un autre couple. Il est alors utile de réduire les décalages temporels entre les différents couples sources-détecteurs lorsque le milieu est excité par une même impulsion.

Afin de corriger un tel décalage peut par exemple calculer tout d'abord le temps de réponse impulsionnel $T\_diff_{sd}$ du signal de diffusion mesuré:

$$T\_diff_{sd} = \frac{\int_0^\infty X_{diff_{sd}}(t)\, t\, dt}{\int_0^\infty X_{diff_{sd}}(t)\, dt}\ .$$

Ainsi, à chaque couple source-détecteur correspond un temps moyen du signal de diffusion $T\_diff_{sd}$. Ce temps moyen quantifie, pour une configuration source-détecteur donnée, la durée moyenne entre l'émission lumineuse et la détection des photons de diffusion par le détecteur.

Lors d'une telle acquisition, on peut retirer le filtre 16 disposé en amont du détecteur 4, de telle sorte que le moyen de détection 4 détecte un signal à la longueur d'onde d'excitation, et non à la longueur d'onde de fluorescence comme ce sera le cas lors des acquisitions de fluorescence.

On peut calculer ensuite le temps moyen théorique du signal diffusion, dans le milieu diffusant, ppelé $\hat{T}\_diff_{sd}$, prédit par le modèle de diffusion choisi (le « chapeau (^) » désigne le modèle, par opposition à la mesure). Par exemple, dans un milieu infini, ce temps moyen est déterminé en divisant la distance entre une source et un détecteur par la vitesse de la lumière dans le milieu. On rappelle que par source et détecteur, on entend ici une extrémité de la fibre d'excitation 10i et une extrémité de la fibre d'émission 12i , les deux extrémités étant fixées sur la sonde 30. Dans notre cas, on calcule $\hat{T}\_diff_{sd}$ de façon numérique. Pour ce faire, on peut résoudre numériquement l'équation suivante :

$$-\vec{\nabla}.\left(D\vec{\nabla}\vec{\nabla}G_s^{(1)}\right) + \mu_a G_s^{(1)} = \delta(\vec{r} - \vec{r}_s) + \frac{G_s^{(0)}}{c}$$

Avec,

G $G_s^{(1)}$ : = moment d'ordre 1 de la fonction de Green exprimant le transfert d'énergie entre la source et le détecteur.

$G_s^{(0)}$ moment d'ordre 0 de la fonction de Green exprimant le transfert d'énergie entre la source et le détecteur.

$\mu_a$ : coefficient d'absorption du milieu diffusant

$c$ : vitesse de la lumière

$D$ : coefficient de diffusion du milieu

$r_s$ : coordonnées de la source, cette dernière pouvant être assimilée à l'extrémité de la fibre d'émission lorsque la source est fibrée.

Finalement, on définit $T\_impulse_{sd}$ comme la différence des deux temps précédemment définis :

$$T\_impulse_{sd} = T\_diff_{sd} - \hat{T}\_diff_{sd}$$

$T\_impulse_{sd}$ représente le temps de réponse impulsionnel du dispositif, c'est-à-dire le temps moyen s'écoulant entre une impulsion de la source et la détection des photons diffusés par le milieu diffusant. Ce temps moyen de réponse impulsionnel quantifie le délai entre l'émission d'une impulsion et la détection d'un signal de diffusion, corrigé de l'estimation du délai de diffusion dans le milieu. Autrement dit, $T\_impulse_{sd}$ quantifie le temps de réponse impulsionnel du dispositif, tandis que T $_{diffsd}$ quantifie le temps de réponse impulsionnel de l'ensemble dispositif et milieu diffusant.

D'une façon générale, on retiendra que T_impulsesd et T_diffsd sont des temps de réponse impulsionnel, quantifiant un délai moyen entre l'émission d'une impulsion et la détection d'un signal de détection.

Le recalage temporel des données mesurées peut être effectué par rapport à un temps de réponse impulsionnel, qui peut être soit T $_{diffsd}$, soit, et de préférence, T $_{impulse\ sd}$, tels que précédemment définis.

L'objectif du recalage temporel est de permettre ensuite de représenter chaque acquisition $X_{sd}$ selon un axe temporel, de telle sorte que, chaque temps de réponse impulsionnel considéré correspondant à la configuration sd a une même coordonnée selon l'axe des temps. Autrement dit, si X'sd désigne une acquisition $X_{sd}$ temporellement recalée par rapport à un temps de réponse impulsionnel, ou décalée de ce temps de réponse, alors :

$$X'_{sd}(t) = X_{sd}(t - T\_impulse_{sd})$$

ou

$$X'_{sd}(t) = X_{sd}(t - T\_diff_{sd})$$

Autrement dit, chaque acquisition $X_{sd}$ est, dans le temps, recalée ou décalée, en fonction du temps de réponse impulsionnel, ou d'une valeur égale à ce temps de réponse impulsionnel, déterminé pour chaque configuration source-détecteur.

On peut également parler de centrage de chaque acquisition $X_{sd}$ selon le temps de réponse impulsionnel correspondant au couple source détecteur sd : en effet, selon l'équation précédente, pour chaque couple source détecteur, $X_{sd}$' (T_impulse $_{sd}$) = 0 (ou $X_{sd}$' (T_diff$_{sd}$) = 0 .

De cette façon, on limite les décalages temporels entre les signaux résolus en temps correspondant à chaque couple source-détecteur.

Dans la suite de la description, on va décrire plus précisément la factorisation de la matrice d'acquisition X, constituée d'une pluralité d'acquisitions $X_{sd}$. Le procédé décrit s'applique indifféremment à une matrice X constituée d'acquisitions $X_{sd}$ recalées temporellement ou non. De préférence, les acquisitions $X_{sd}$ sont recalées temporellement selon l'équation précédente.

La figure 4 est un exemple d'une superposition d'un jeu de 24 mesures $X_{sd}$ de fluorescence, pour 6 sources et 4 détecteurs. Les acquisitions sont recalées, comme expliqué ci-dessus, par rapport au temps moyen de la mesure de diffusion.

Il reste des décalages entre les positions temporelles des différents pics, dus aux positions relatives des couples source-détecteurs et des marqueurs fluorescents : le trajet entre l'extrémité d'une fibre d'excitation et un marqueur n'est pas le même que celui entre l'extrémité d'une autre fibre d'excitation et le même marqueur, et le problème est le même avec les différentes positions des détecteurs (ou des extrémités des fibres de détection) par rapport à ce même marqueur.

On explique maintenant comment on exploite ces données, de préférence corrigées ou recentrées, en vue de réaliser une image des marqueurs fluorescents dans le milieu. L'étape (optionnelle) précédente permet de recentrer les données sans s'affranchir de l'autofluorescence, ou, plus généralement, des signaux parasites, tandis que les étapes suivantes permettent de séparer ces composantes.

Dans la suite de la description, on suppose que chaque acquisition est une acquisition corrigée en temps. Mais une telle correction n'est pas toujours à mettre en oeuvre.

Considérons une acquisition de fluorescence $X_{sd}$ (c'est-à-dire un ensemble de données résultant d'un échantillonnage d'une courbe de fluorescence telle que l'une de celles des figures 3A- 3B) sur un milieu diffusant contenant un type de marqueur fluorescent.

Cette mesure est la superposition de deux distributions temporelles distinctes :

- $s_1$ : distribution temporelle des photons d'intérêt émis par les marqueurs fluorescents, dite composante temporelle de fluorescence ou composante d'intérêt,
- $s_2$ :distribution temporelle de photons dits parasite, incluant l'autofluorescence et/ou de diffusion, dite composante temporelle parasite.

Si on appelle $a_1$ et $a_2$ les quantités respectives de ces deux distributions, l'acquisition $X_{sd}$ peut s'écrire comme la combinaison linéaire suivante des 2 distributions $s_1$ et $s_2$:

$$X_{sd} = a_1 s_1 + a_2 s_2 = (a_1\ a_2) \times \begin{pmatrix} s_{1,1} \dots s_{1,N_T} \\ s_{2,1} \dots s_{2,N_T} \end{pmatrix} = AS \qquad (1)$$

où chaque terme $S_{p,t}$ de la matrice S est la valeur du profil de la composante p correspondant à l'intervalle temporel t. Chaque intervalle temporel t correspond généralement à la fréquence de l'échantillonage temporel mis en oeuvre lors de l'acquisition $X_{sd}$.

Ainsi :

- $s_{1,i}$ désigne la ième valeur de la distribution $s_1$,
- $s_{2,i}$ désigne la ième valeur de la distribution $s_2$.

Selon cette méthode on cherche à trouver des matrices A et S à coefficients non-négatifs, dont le produit est le plus proche possible de la matrice $X_{sd}$.

On peut généraliser cet exemple de traitement de données à une série *X* de $N_x$ acquisitions. X est alors une matrice non-négative $X \in R^{N_x \times N_t}$, dont chaque terme $X_{x,t}$ correspond à la valeur de l'acquisition $X_x$ dans le canal temporel t. Comme précédemment indiqué, l'indice x peut être remplacé par l'indice sd, ce dernier représentant la position d'une source s et d'un détecteur d. $N_x$ représente alors le nombre de couples source-détecteur considérés.

On cherche alors à trouver les matrices non-négatives $A \in R^{N_x \times N_p}$ et $S \in R^{N_x \times N_p}$ telles que .

$$X \approx AS \qquad (2)$$

Par matrice non-négative on entend une matrice dont tous les éléments sont non-négatifs, et Np représente le nombre de composantes temporelles, ou de distributions temporelles $s_i$, recherchées.

A est dite matrice de poids. Un élément $a_{sd,p}$ ($\geq 0$) de cette matrice représente la contribution, dans une acquisition de fluorescence $X_{sd}$, d'une composante temporelle de fluorescence ou d'une composante temporelle de diffusion et/ou d'autofluorescence, ces composantes étant désignées par l'indice p (par exemple p = 1 désignant la composante de fluorescence et p = 2 désignant la composante parasite).

S est la matrice des composantes de X à séparer les unes des autres (dans cet exemple, composante de fluorescence ou composante parasite), chaque ligne de cette matrice, notée Sp, comportant des données discrétisées représentant un profil temporel de la composante p.

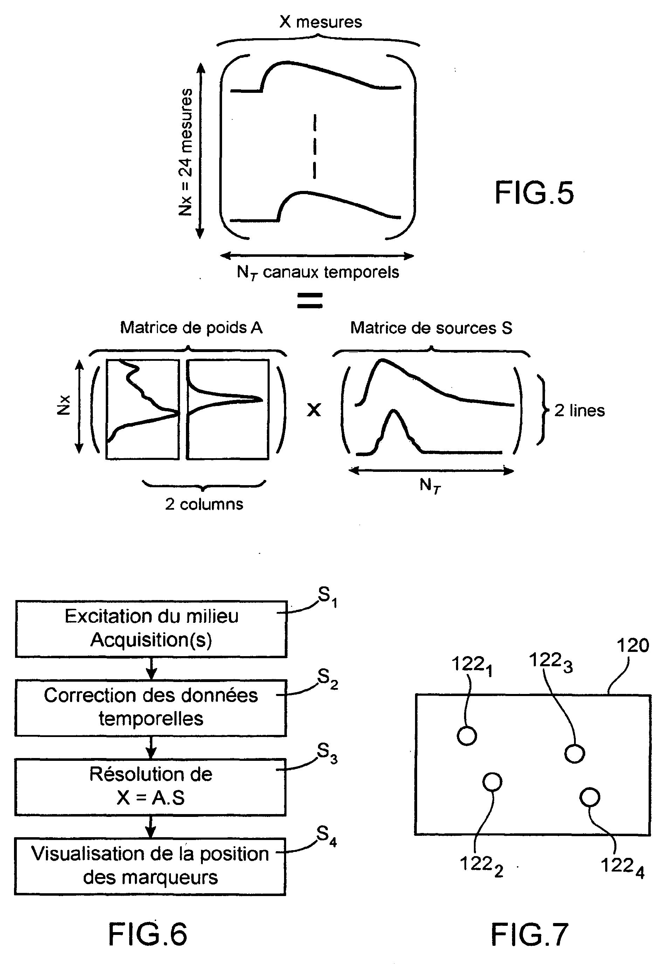

Si on considère uniquement 2 composantes temporelles (p = 2), cette équation peut être illustrée selon le schéma de la figure 5 pour le cas de 24 mesures.

Le problème est ensuite de trouver des matrices particulières A et S pour satisfaire l'équation ci-dessus.

Pour cela, on cherche à minimiser une fonction de coût F, par exemple le carré de la distance euclidienne entre *X* et *AS,* ou une adaptation de la divergence de Kullback-Leibler. On peut également utiliser une autre expression, comprenant le carré de ladite distance euclidienne, détaillée dans la suite de la description.

Si on considère la distance euclidienne entre la matrice X et le produit des deux matrices A et S, alors on cherche à minimiser la fonction de coût F:

$$\| X - AS \|^2$$

avec A$\geq$0 et S$\geq$0.

La solution est obtenue de manière approchée, par itérations.

Différentes méthodes peuvent être utilisées pour minimiser la fonction de coût. On peut citer les lois de mise à jour multiplicatives introduites par Lee et Seung dans l'article « Learning the parts of objects by non négative matrix factorization», paru dans Nature, pages 788-791, 1999. Selon cette technique la procédure générale d'optimisation peut donc s'écrire :

- 1. initialisation de A et S par des valeurs aléatoires positives,
- 2. mise à jour de A

- 3. mise à jour de S
- 4. recommencer les étapes 2 et 3 jusqu'à ce que la fonction de coût F, ou sa variation entre deux itérations successives, atteigne un critère de convergence, ou jusqu'à un certain nombre d'itérations.

Dans le cas de $N_x$ acquisitions (par exemple) les 24 mesures obtenues avec le dispositif des figures 2A et 2B), chacune étant discrétisée suivant $N_t$ points dans le temps, on a donc :

$$X = \begin{pmatrix} x_{11} & \cdots & x_{1.Nt} \\ \vdots & \ddots & \vdots \\ x_{Nx.1} & \cdots & x_{Nx.Nt} \end{pmatrix} = \begin{pmatrix} a_{11} & a_{12} \\ \vdots & \vdots \\ a_{Nx.1} & a_{Nx,2} \end{pmatrix} \begin{pmatrix} s_{11} & & s_{1.Nt} \\ s_{21} & & s_{2.Nt} \end{pmatrix}$$

Si l'indice p = 1 désigne la composante temporelle de fluorescence, et que l'indice p = 2 désigne la composante temporelle parasite, alors le produit $a_{x,1} * S_1$ correspond à l'histogramme de la composante temporelle de fluorescence correspondant à l'acquisition $X_x$.

Cette composante temporelle de fluorescence peut être notée $I_x$ (ou $I_{sd}$ lorsque l'indice désigne un couple source-détecteur). Cette composante $I_x$ correspond à l'acquisition $X_x$ corrigée de sa composante parasite. Autrement dit, $I_x$ représente la partie de $X_x$ réellement due aux marqueurs fluorescents, d'où la dénomination composante d'intérêt. On peut également écrire que Ix représente le signal "net" de fluorescence, alors que Xx représente le signal "brut" (i-e non corrigé) de fluorescence.

Le produit $a_{x,2} * S_2$ correspond, lui, à la composante temporelle parasite de l'acquisition $X_x$

On retrouve bien l'équation déjà présentée ci-dessus : $a_{x,i} * S_1 + a_{x,2} * S_{2 \approx} X_x$.

On voit donc qu'à partir des matrices A et S, on obtient une composante temporelle, désignée p, d'une acquisition $X_x$, en multipliant les termes de la ligne Sp par le coefficient (ou poids) $a_{x,p}$. Selon la valeur de p, cette composante peut être une composante d'intérêt (composante de fluorescence dans cet exemple) ou une composante parasite.

D'une façon plus générale, cette composante p est obtenue en multipliant des termes de la matrice S, relatifs à cette même composante, par le terme $a_{x,p}$, de la matrice A, représentant le poids de la contribution p dans l'histogramme $X_x$.

On a donné en figure 5 l'exemple imagé du produit d'une matrice S (pour une acquisition avec deux sources fluorescentes) avec un tableau A afin d'obtenir le tableau X.

On a donné ci-dessus un exemple de fonction de coût F à minimiser. Mais celle-ci peut avoir différentes formes. On peut aussi utiliser, de façon avantageuse le carré de la distance euclidienne entre X et AS, plus un terme de contrainte :

$$F = \|X - AS\|^2 + \alpha \|S - S_0\|^2 \qquad (1)$$

où $S_0$ est la matrice S initiale, comme expliqué ci-dessous, et $\alpha$ un paramètre dit de régularisation. Ce dernier permet de contraindre plus ou moins la solution S à rester proche de l'initialisation choisie $S_0$. Le fait d'utiliser une telle fonction de coût permet d'accroître la robustesse de la méthode. Cela peut également permettre d'accroître la vitesse de convergence

Un exemple d'algorithme pouvant être utilisé pour calculer le couple de matrices A et S dont la produit représente au mieux les données initiales *X* est alors, là encore, un algorithme itératif. Le nombre d'itérations $N_{it}$ est déterminé soit de façon empirique, soit par un critère d'arrêt choisi, comme précédemment évoqué.

L'algorithme comprend les étapes suivantes :

1. Initialisation des matrices A et S avec des valeurs strictement positives. On peut choisir effectuer l'initialisation de S avec des modèles de sources mesurés expérimentalement ou tirés de la littérature.
2. Choix du paramètre de régularisation $\alpha$ .
Le choix se fait de façon empirique suivant la confiance que l'on donne à l'initialisation (plus $\alpha$ est grand, plus la matrice S calculée est contrainte à être proche de l'initialisation).

Il y a ensuite mise à jour de A et S, à tour de rôle pendant $N_{it}$ itérations.

Plusieurs méthodes peuvent être mises en oeuvre pour cette mise à jour. Par exemple, on peut citer une descente de gradient alternativement sur A et S (appelée « ALS » pour « Alternative Least Square ») ou les lois de mises à jour multiplicatives indiquées par Lee et Seung dans l'article déjà ci-dessus.

En s'inspirant de ces dernières, et en définissant $X^T$ la transposée d'une matrice X, si la fonction de coût à minimiser est $F = \|X\text{-}AS\|^2 + \alpha \|S\text{-}S_0\|^2$, ce qui constitue la variante préférée, chaque itération, comprend les mises à jour suivantes pour A et S :

$$S_{i+1} \leftarrow S_i \frac{\left(A_i^T X + \alpha S_0\right)}{\left(A_i^T A_i S + \alpha S_i\right)}$$

puis :

$$A_{i+1} \leftarrow A_i \frac{\left(XS_{i+1}^{\phantom{i}T}\right)}{\left(A_i S_{i+1} S_{i+1}^{\phantom{i}T}\right)}$$

Ces lois de mise à jour particulières ont été développées par les inventeurs.

On peut choisir une fonction de coût F plus classique, précédemment évoquée, telle que $F = \|X - AS\|^2$

Dans ce cas, à chaque itération, les lois de mise à jour sont celles décrites par Lee et Seung :

$$S_{i+1} \leftarrow S_i \frac{\left(A_i^T X\right)}{\left(A_i^T A_i S\right)}$$

Puis

$$A_{i+1} \leftarrow A_i \frac{\left(XS_{i+1}^{\phantom{i}T}\right)}{\left(A_i S_{i+1} S_{i+1}^{\phantom{i}T}\right)}$$

A partir des données des matrices A et S, on peut extraire, pour chaque acquisition $X_x$, une composante de fluorescence, ou composante d'intérêt, due aux marqueurs, et notée Ix.

On peut alors mettre en oeuvre une méthode de reconstruction basée sur le modèle de l'équation de la diffusion, par exemple telle que décrite dans J.Boutet et al. dans « Bimodal ultrasound and fluorescence approach for prostate cancer diagnosis", J. Biomed. Opt. 14, 064001 (2009).

Pour chaque courbe temporelle obtenue, on peut calculer les moments d'ordre 0 et 1 du signal de fluorescence corrigé $I_x(t)$ :

$$M_x^0 = \int_0^\infty I_x(t).dt \qquad\qquad (2)$$

$$M_x^1 = \int_0^\infty I_x(t).t.dt \qquad\qquad (3)$$

L'obtention de tels moments permet de déterminer la position des fluorophores, selon des techniques telles que par exemple décrites dans l'article de J.Boutet et al. cité ci-dessus, ou encore dans le document "Méthodes optiques résolues en temps pour la tomographie de fluorescence dans les milieux diffusants",Thèse de Aurélie Laidevant soutenue le 5 octobre 2006 à l'Université Joseph Fourier - Grenoble - France ou encore dans la demande EP10179184 du 24 septembre 2010.

Afin de tester les différentes étapes d'un procédé selon l'invention, on a pu réaliser différents tests.

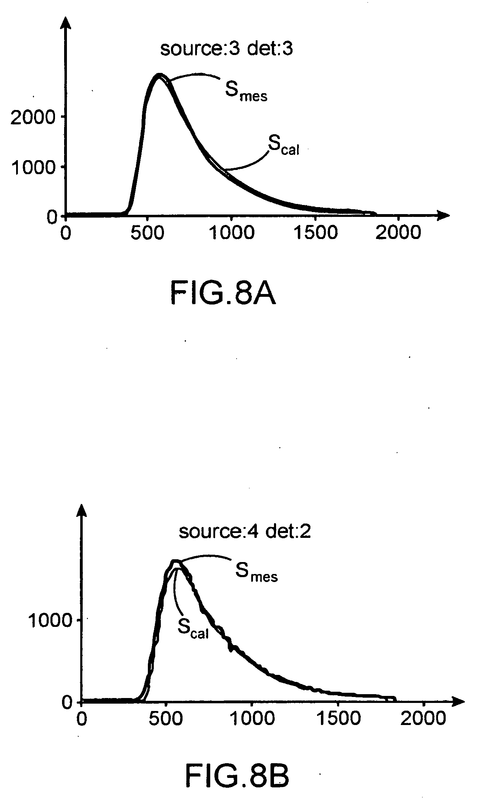

Pour chaque couple source-détecteur, les comparaisons entre les signaux temporels mesurés (moins le signal parasite de diffusion) et les signaux calculés par le procédé selon l'invention ont été réalisées en utilisant un fantôme de fluorescence. Des exemples de résultats sont donnés sur les figures 8A - 8B (figure 8A : pour le couple source 3, détecteur 3, figure 8B : pour le couple source 4, détecteur 2). Sur ces figures, le signal mesuré correspond à :

- un premier signal mesuré lorsqu'une inclusion fluorescente est présente dans le fantôme,
- auquel on a soustrait un second signal, ce dernier étant mesuré lorsque l'inclusion fluorescente est retirée et représentant donc la composante parasite.

Sur ces figures, le signal mesuré est identifié par $S_{mes}$ et le signal calculé par $S_{cal}$ (composante fluorescente du premier signal décrit dans l'alinéa précédent, déterminée selon le procédé objet de l'invention). Les deux signaux sont pratiquement confondus

La soustraction permet d'évaluer la composante de fluorescence du premier signal.

Dans toutes les comparaisons effectuées, dont deux exemples sont en figures 8A - 8B, on a une composante de fluorescence expérimentale (signal mesuré décrit précédemment) et une composante calculée (signal calculé décrit précédemment). Dans la plupart des cas, l'accord entre les valeurs de signal mesuré et le signal calculé est très bon.

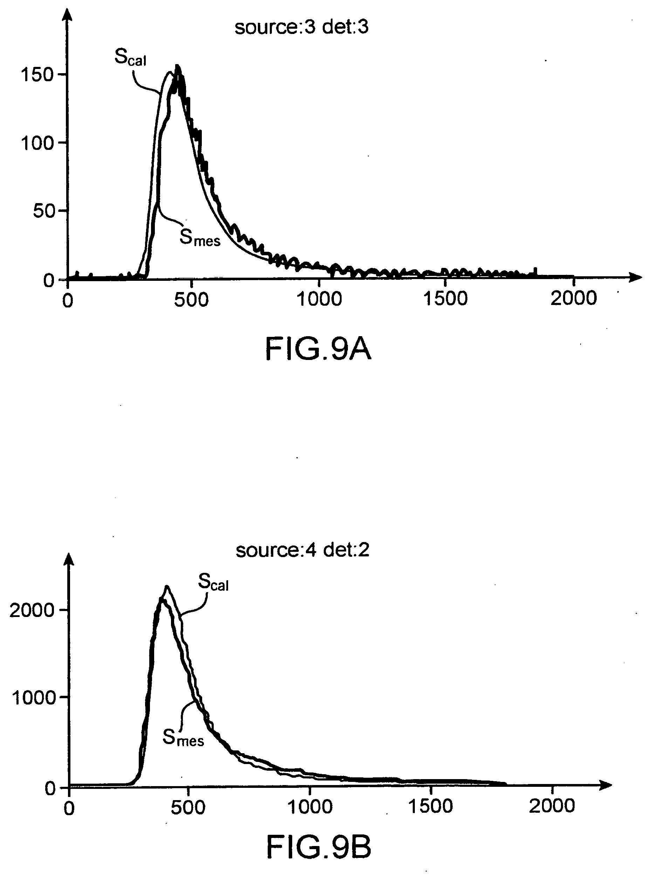

De même, pour chaque couple source-détecteur, des comparaisons ont été effectuées entre les signaux parasites mesurés et les signaux parasites calculés par le procédé selon l'invention. Deux exemples en sont donnés sur les figures 9A - 9B (figure 9A : pour le couple source 3, détecteur 3 ; figure 9B : pour le couple source 4, détecteur 2). Sur chacune de ces figures .

- le signal mesuré $S_{mes}$ correspond au signal mesuré lorsque l'inclusion fluorescente est retirée. Il représente la composante parasite.
- le signal calculé $S_{cal}$ est la composante parasite d'un signal mesuré lorsque l'inclusion fluorescente est placée dans le fantôme. Cette composante parasite est déterminée selon le procédé objet de l'invention.

Dans certains cas, et en particulier sur les figures 9A - 9B, les deux signaux sont pratiquement confondus.

Cela permet d'obtenir une composante parasite expérimentale (signal mesuré décrit précédemment) et calculée (signal calculé décrit précédemment ) .

On a pu constater, dans la plupart des cas un bon accord entre les mesures et le calcul, ce qui permet une validation expérimentale de la méthode.

Une comparaison avec les résultats obtenus avec d'autres méthodes a été réalisée.

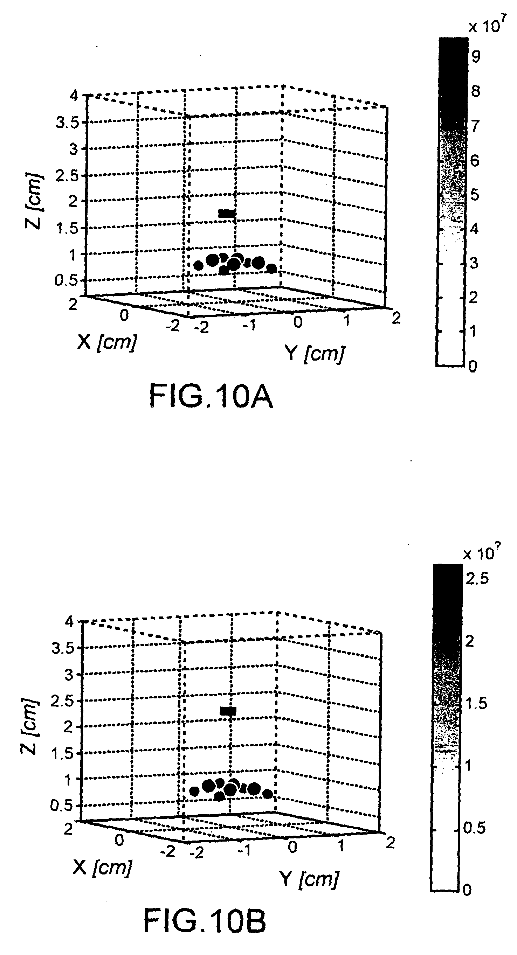

Ainsi les figures 10A et 10B montrent respectivement une comparaison entre une localisation par un procédé sans correction puis par un procédé selon l'invention, donc avec correction. Dans cette expérience, la position réelle du marqueur fluorescent selon l'axe Z, par rapport à la limite du milieu diffusant est z = 2 cm. En appliquant une technique classique et sans correction de l'autofluorescence on obtient z = 1.7 cm (figure 10A). Avec correction expérimentale, on obtient : z = 2.12 cm (figure 10B).

Lors de la correction expérimentale, on détermine expérimentalement la composante parasite (mesure sans inclusion fluorescente) et on la soustrait des mesures réalisées avec l'inclusion fluorescente

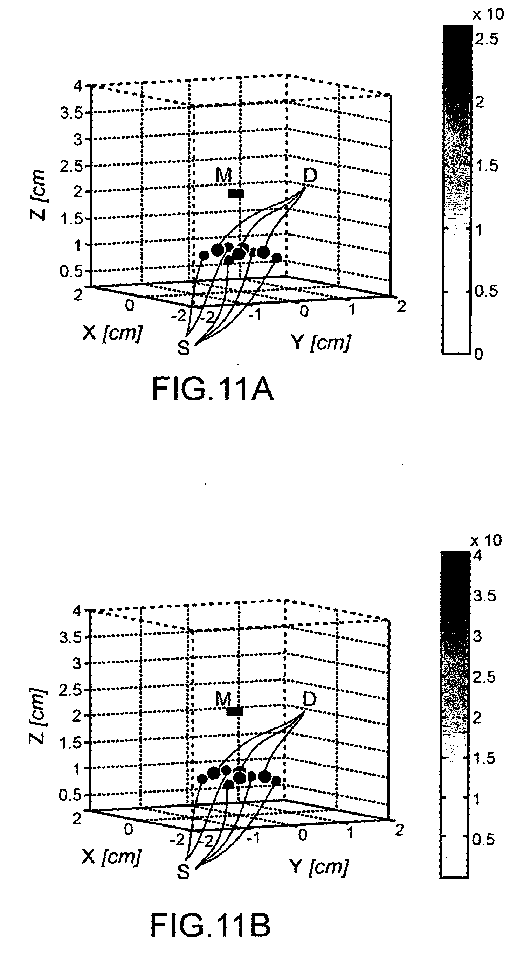

La comparaison des figures 10A et 10B montre que, sans correction de l'autofluorescence, l'inclusion est reconstruite à une position trop proche de la sonde (comme sur les figures 11A-11B, les sources S sont identifiées sur ces figures 10 par les disques noirs de faible diamètre et les détecteurs D par des disques noirs de plus grand diamètre).

Les figures 11A et 11B montrent respectivement une comparaison entre une comparaison dite « expérimentale » , c'est-à-dire réalisée en soustrayant une mesure sur un fantôme réalisée sans l'inclusion fluorescente d'une mesure sur fantôme réalisée avec l'inclusion fluorescente et une comparaison par un procédé selon l'invention.

Plus précisément, la figure 11B montre un résultat obtenu avec correction par la méthode de séparation des composantes selon le procédé précédemment décrit en utilisant une fonction de coût basée sur la distance euclidienne pour laquelle on obtient z=2.19 cm. Les résultats de la correction expérimentale (figure 11A) et de la correction selon l'invention (figure 11B) sont similaires.

Des étapes d'un procédé selon l'invention sont représentées en figure 6 :

- dans une étape S1, on réalise une ou plusieurs acquisitions par excitation du milieu diffusant, par un faisceau laser fonctionnant en impulsions; il en résulte par exemple une ou plusieurs acquisitions,
- lors de l'étape S2, on forme la matrice X, avec, pour chaque ligne, des données numérisées et échantillonnées d'une courbe de signal de fluorescence pour un couple (source, détecteur) ou un couple (fibre d'excitation, fibre de détection) donné,
- dans une étape S3, optionnelle, on réalise une correction des données temporelles mesurées précédemment pour le signal de diffusion,
- l'équation $X \approx A.S$ peut ensuite être résolue, de manière itérative comme expliqué ci-dessus (étape S4),
- on peut ensuite déterminer les acquisitions I correspondant à la composante recherchée, en l'occurrence la composante de fluorescence des marqueurs,

- on peut alors procéder à une représentation graphique du ou des marqueurs fluorescents (étape S5).

Lors d'une mise en oeuvre d'un procédé selon l'invention sur un patient, l'étape d'excitation par les sources de rayonnement peut être précédée par une étape d'injection d'un marqueur fluorescent dans le milieu dont une zone est à étudier. On cherche à localiser ce ou ce(s) marqueur(s) fluorescent(s) dans ce milieu diffusant.

Dans toute la description ci-dessus, les acquisitions Xx étaient représentées sous la forme de matrice ligne. Mais l'invention s'applique aussi lorsque ces acquisitions sont représentées sous forme de vecteurs-colonnes. Dans ce cas, chaque colonne x de la matrice X correspond à une acquisition, et on obtient la contribution de la composante p au vecteur $X_x$ en effectuant le produit du terme vecteur Sp (pième colonne de la matrice S) par le coefficient $a_{p,x}$. La factorisation de la matrice X en matrices non négatives permet alors de déterminer S et A telles que X = S A telles que .

- X représente la matrice des acquisitions, de dimension (Nt, Nx),
- S représente la matrice des profils des contributions, de dimension (Nt, Np),
- A représente la matrice des poids, de dimension (Np, Nx).

Bien que dans cette description, on ait considéré deux composantes (une composante de fluorescente et une composante parasite), l'invention s'applique à un nombre plus important de composantes, par exemple plusieurs composantes de fluorescence et une composante parasite, ou seulement plusieurs composantes de fluorescence lorsque la composante parasite s'avère être négligeable.

L'invention s'applique à d'autres cas que celui de marqueurs fluorescents.

En effet, l'injection de marqueurs fluorescents peut présenter certaines contraintes.

Il est parfois préférable de s'en affranchir et de chercher à localiser, dans un milieu 120 (figure 7), non pas des marqueurs fluorescents, mais des zones $122_1$, $122_2$, $122_3$, $122_4$ présentant des propriétés optiques différentes de celles du milieu diffusant 120 environnant. En particulier, on peut rechercher des zones, dites zones d'intérêt, ces zones d'intérêt étant alors considérées comme des marqueurs optiques et présentant :

- un coefficient d'absorption et ou de diffusion différent de celui du milieu diffusant (dans ce cas on détecte un rayonnement diffusé à travers le tissu, à la longueur d'onde d'excitation,
- et/ou un coefficient de diffusion différent de celui du milieu diffusant environnant ; dans ce cas, on détecte un rayonnement diffusé différemment par les marqueurs et par le milieu diffusant.

Le cas de zones absorbantes, qui peuvent être considéré comme marqueurs, est celui de certaines tumeurs cancéreuses.

Un procédé selon l'invention comprend alors des étapes identiques ou similaires à celui précédemment décrit dans le cadre de la fluorescence, à savoir :

- l'éclairement d'un milieu diffusant par une lumière d'excitation en impulsions, de préférence dans l'infra-rouge ou dans le rouge, afin de limiter l'absorption dans les tissus,
- la détection du signal diffusé résolu en temps par le milieu, à la longueur d'onde d'excitation, et la réalisation d'acquisitions prenant la forme d'histogrammes temporels $X_x$ du temps d'arrivée des photons, issus du milieu, sur le détecteur,
- la constitution d'une matrice X rassemblant une ou plusieurs ($N_x$) acquisition $X_x$,
- la décomposition de la matrice X en un produit de deux matrices non négatives A et S,
- la détermination d'une composante d'intérêt, ou composante utile, en multipliant des termes de la matrice A par des termes de la matrice S, par exemple en multipliant un coefficient $a_{x,p}$ par les termes de la matrice S représentatifs de la contribution p.

La composante utile $I_x$ à l'acquisition $X_x$ représente alors le signal diffusé par les zones d'intérêt à la longueur d'onde d'excitation $\lambda_{ex}$. On peut alors déterminer, par reconstruction, la position de ces zones dans le milieu.

Dans tous les cas un rayonnement d'excitation a une longueur d'onde inférieure à une ou plusieurs longueurs d'one détectées, quel que soit le type d'interaction entre le rayonnement d'excitation et le milieu, contenant un ou plusieurs marqueurs tels que défini ci-dessus :

- si le marqueur est un marqueur fluorescent, la longueur d'onde de fluorescence est supérieure à la longueur d'onde du rayonnement d'excitation,
- si le marqueur a un coefficient d'absorption et/ou de diffusion différent de celui du milieu diffusant, alors le rayonnement détecté a une longueur d'onde supérieure ou égale au rayonnement d'excitation.

**Revendications**

1. Procédé de localisation d'au moins un marqueur optique (22, $122_1$, $122_2$, $122_3$, $122_4$) dans un milieu diffusant, ledit marqueur ayant au moins une propriété optique différente du milieu diffusant, procédé dans lequel :

   a) on réalise au moins une acquisition d'un signal optique caractéristique du milieu (20, 120) et d'un ou plusieurs desdits marqueurs (22, $122_1$, $122_2$, $122_3$, $122_4$), par une interaction du milieu avec le rayonnement d'une source de rayonnement (8) en impulsions, chaque acquisition comportant d'une part une ou plusieurs composantes temporelles d'intérêt, due à un ou plusieurs marqueurs, d'autre part une composante temporelle parasite, due à une partie du milieu autre que les marqueurs,
   b) on forme, à partir des données de signal optique d'au moins une desdites acquisitions, une matrice multidimensionnelle X,
   c) on traite les données de ladite matrice X par factorisation de cette matrice en un produit de, seulement, deux matrices multidimensionnelles non négatives A et S,
   d) on extrait la contribution, au signal optique, d'au moins une desdites composantes temporelles, à partir des données contenues dans les matrices A et S.

2. Procédé selon la revendication 1, comportant en outre une étape de calcul de la position d'au moins un marqueur optique dans le milieu diffusant et/ou une représentation graphique de la répartition d'au moins un tel marqueur dans le milieu diffusant, à partir d'au moins une desdites composantes temporelles.

3. Procédé selon l'une des revendications précédentes, dans lequel le marqueur optique :

   - comporte au moins un marqueur fluorescent, l'excitation du milieu étant alors réalisée à au moins une longueur d'onde d'excitation d'au moins un marqueur fluorescent, le signal optique résultant de l'excitation étant à au moins une longueur d'onde de fluorescence d'au moins un marqueur fluorescent, et dans lequel on extrait la contribution de fluorescence due à au moins un marqueur fluorescent à partir des termes des matrices A et S,
   - ou comporte au moins une zone présentant au moins un coefficient d'absorption et/ou de diffusion différent de celui du milieu diffusant environnant (20, 120), le signal optique résultant de la diffusion du signal d'excitation dans le milieu diffusant (20, 120) et dans lequel on extrait la contribution due à au moins une zone à coefficient d'absorption différent de celui du milieu diffusant.

4. Procédé selon l'une des revendications 1 à 3, dans lequel, avant formation de ladite matrice multidimensionnelle, on corrige les données de signal optique de chaque acquisition par recentrage des données temporelles dans une plage temporelle par rapport à un temps de réponse impulsionnel.

5. Procédé selon l'une des revendications 1 à 4, dans lequel, lors de l'étape c), on détermine A et S par minimisation d'une fonction de coût.

6. Procédé selon la revendication 5, dans lequel, la fonction de coût comporte :

   - la distance $\|X\text{-}AS\|^2$ entre l'image X et le produit A.S ;
   - ou la fonction :

$$F = \left\| X - AS \right\|^2 + \alpha \left\| S - S_0 \right\|^2$$

   Où $S_0$ est la matrice initiale et $\alpha$ un coefficient dit de régularisation.

7. Procédé selon l'une des revendications 1 à 6, dans lequel l'étape c) est réalisée par k itérations, des matrices $A_{l+1}$ et $S_{l+1}$, obtenus lors de l'itération d'ordre 1+1 étant déterminées à partir des tableaux $A_l$ et $S_l$ obtenus lors de l'itération d'ordre 1.

8. Procédé selon la revendication 7, dans lequel le nombre d'itération est déterminé en fonction des fluctuations des matrices A et S, ou de façon automatique, en fonction de fluctuations de la fonction de coût au cours de 2 ou plusieurs itérations successives.

9. Procédé selon l'une des revendications 1 à 8, dans lequel le rayonnement d'excitation a un spectre au moins en partie dans l'infra rouge et/ou le signal optique est détecté à au moins une longueur d'onde supérieure à 600 nm.

10. Dispositif d'identification d'au moins un marqueur optique dans un milieu diffusant, comportant :

a) une source de rayonnement en impulsions (2, 4) pour produire un faisceau d'excitation d'au moins un marqueur optique dans ledit milieu,

b) des moyens (6, 8) pour réaliser au moins une acquisition d'un signal optique, caractéristique du milieu et d'un ou plusieurs marqueurs, chaque acquisition comportant, d'une part, une ou plusieurs composantes temporelles d'intérêt, due à un ou plusieurs marqueurs, et d'autre part, une composante temporelle parasite, due à une partie du milieu autre que les marqueurs,

c) des moyens pour former, à partir des données de signal optique d'au moins une desdites acquisitions, une matrice multidimensionnelle X,

d) des moyens (24, 27, 28) pour traiter les données de ladite matrice en un produit de, seulement, deux matrices multidimensionnelles non négatives A et S,

e) des moyens (24, 27, 28) pour extraire la contribution, au signal optique, d'au moins une desdites composantes temporelles, à partir des données contenues dans les matrices A et S.

11. Dispositif selon la revendication 10, comportant en outre des moyens pour calculer la position d'au moins un marqueur optique dans le milieu diffusant et/ou une représentation graphique de la répartition d'au moins un tel marqueur dans le milieu diffusant, à partir d'au moins une desdites composantes temporelles.

12. Dispositif selon l'une des revendications 10 ou 11, comportant en outre des moyens, pour, avant formation de ladite matrice multidimensionnelle, corriger les données de signal optique de chaque acquisition par recentrage des données temporelles dans une plage temporelle autour d'un temps moyen du signal optique.

13. Dispositif selon l'une des revendications 10 à 12, comportant dés moyens pour déterminer A et S par minimisation d'une fonction de coût.

14. Dispositif selon l'une des revendications 10 à 13, dans lequel la source de rayonnement en impulsions (2, 4) permet de produire un faisceau d'excitation au moins en partie dans l'infra rouge.

15. Dispositif selon l'une des revendications 10 à 14, dans lequel les moyens (6, 8) pour réaliser au moins une acquisition d'un signal optique comportant des moyens pour détecter un rayonnement à au moins une longueur d'onde supérieure à 600 nm.

**Patentansprüche**

1. Verfahren zum Lokalisieren mindestens eines optischen Markers (22, $122_1$, $122_2$, $122_3$, $122_4$) in einem streuenden Medium, wobei der Marker mindestens eine von dem streuenden Medium verschiedene optische Eigenschaft aufweist, wobei:

a) wenigstens eine Erfassung von einem für das Medium (20, 120) und für einen oder mehrere der Marker (22, $122_1$, $122_2$, $122_3$, $122_4$) charakteristischen optischen Signal aufgrund einer Wechselwirkung des Mediums mit der Strahlung einer gepulsten Strahlungsquelle (8) umgesetzt wird, wobei jede Erfassung einerseits eine oder mehrere zeitliche Komponenten von Interesse, die von einem oder mehreren Markern herrührt bzw. herrühren, andererseits eine parasitäre zeitliche Komponente umfasst, die von einem anderen Teil des Mediums als den Markern herrührt,

b) auf Grundlage von Daten des optischen Signals von wenigstens einer der Erfassungen eine mehrdimensionale Matrix X gebildet wird,

c) die Daten der Matrix X durch Faktorisierung dieser Matrix in ein Produkt aus nur zwei nicht-negativen mehrdimensionalen Matrizen A und S verarbeitet werden,

d) aus dem optischen Signal der Beitrag von mindestens einer der zeitlichen Komponenten extrahiert wird, ausgehend von den Daten, die in den Matrizen A und S enthalten sind.

2. Verfahren nach Anspruch 1, ferner umfassend einen Schritt der Berechnung der Position von wenigstens einem optischen Marker in dem streuenden Medium und/oder einer graphischen Darstellung der Verteilung von mindestens einem solchen Marker in dem streuenden Medium, ausgehend von wenigstens einer der zeitlichen Komponenten.

3. Verfahren nach einem der vorhergehenden Ansprüche, wobei der optische Marker:

- mindestens einen fluoreszierenden Marker umfasst, wobei die Anregung des Mediums bei mindestens einer Anregungswellenlänge von mindestens einem fluoreszierenden Marker umgesetzt wird, wobei das aus der Anregung resultierende optische Signal bei wenigstens einer Fluoreszenz-Wellenlänge von mindestens einem fluoreszierenden Marker ist, und wobei der von wenigstens einem fluoreszierenden Marker herrührende Fluoreszenz-Beitrag ausgehend von Termen der Matrizen A und S extrahiert wird,
- oder mindestens einen Bereich umfasst, der wenigstens einen Absorptions- oder/und Diffusionskoeffizienten aufweist, der verschieden von demjenigen des umgebenden streuenden Mediums (20, 120) ist, wobei das optische Signal aus der Streuung des Anregungssignals im streuenden Medium (20, 120) resultiert, und wobei der Beitrag extrahiert wird, der von wenigstens einem Bereich mit einem Absorptionskoeffizienten herrührt, der von dem des streuenden Mediums verschieden ist.

4. Verfahren nach einem der Ansprüche 1 bis 3, wobei vor der Bildung der mehrdimensionalen Matrix, die Daten des optischen Signals jeder Erfassung durch Rezentrierung der zeitlichen Daten in einem Zeitbereich in Bezug auf eine Impulsantwort-Zeit korrigiert werden.

5. Verfahren nach einem der Ansprüche 1 bis 4, wobei bei dem Schritt c) A und S durch Minimieren einer Kostenfunktion bestimmt werden.

6. Verfahren nach Anspruch 5, wobei die Kostenfunktion umfasst :

- den Abstand $\|X - AS\|^2$ zwischen dem Bild X und dem Produkt A.S ;
- oder die Funktion:

$$F = \|X - AS\|^2 + \alpha \|S - S_0\|^2$$

wobei $S_0$ die Ausgangsmatrix und $\alpha$ ein Koeffizient ist, der Regularisierungskoeffizient genannt wird.

7. Verfahren nach einem der Ansprüche 1 bis 6, wobei der Schritt c) durch k Iterationen durchgeführt, wobei die bei der Iteration der Ordnung l+1 erhaltenen Matrizen $A_{l+1}$ und $S_{l+1}$ ausgehen von Matrizen A und $S_l$ bestimmt werden, die bei der Iteration der Ordnung l erhalten wurden.

8. Verfahren nach Anspruch 7, wobei die Anzahl von Iterationen in Abhängigkeit von Fluktuationen der Matrizen A und S, oder automatisch in Abhängigkeit von Fluktuationen der Kostenfunktion bei zwei oder mehr aufeinander folgenden Iterationen bestimmt wird.

9. Verfahren nach einem der Ansprüche 1 bis 8, wobei die Anregungsstrahlung ein Spektrum zumindest teilweise im infraroten Bereich aufweist, oder/und das optisches Signal bei wenigstens einer Wellenlänge größer als 600 nm erfasst wird.

10. Vorrichtung zum Identifizieren von mindestens einem optischen Marker in einem streuenden Medium, umfassend:

a) eine gepulste Strahlungsquelle (2, 4) zum Erzeugen eines Strahls zur Anregung von mindestens einem optischen Marker in dem Medium,
b) Mittel (6, 8) zum Durchführen von mindestens einer Erfassung eines optischen Signals, das charakteristisch für das Medium und einen oder mehrere Marker ist, wobei jede Erfassung einerseits eine oder mehrere zeitliche Komponenten von Interesse, die von einem oder mehreren Markern herrührt bzw. herrühren, andererseits eine parasitäre zeitliche Komponente umfasst, die von einem anderen Teil des Mediums als den Markern herrührt,
c) Mittel zum Bilden einer mehrdimensionalen Matrix X, ausgehend von Daten von dem optischen Signal wenigstens einer der Erfassungen,
d) Mittel (24, 27, 28), um die Daten der Matrix in ein Produkt aus nur zwei nicht-negativen mehrdimensionalen Matrizen A und S zu verarbeiten,
e) Mittel (24, 27, 28), um aus dem optischen Signal den Beitrag von mindestens einer der zeitlichen Komponenten zu extrahieren, ausgehend von den Daten, die in den Matrizen A und S enthalten sind.

**11.** Vorrichtung nach Anspruch 10, ferner umfassend Mittel zum Berechnen der Position von wenigstens einem optischen Marker in dem streuenden Medium und/oder einer graphischen Darstellung der Verteilung von mindestens einem solchen Marker in dem streuenden Medium, ausgehend von wenigstens einer der zeitlichen Komponenten.

**12.** Vorrichtung nach einem der Ansprüche 10 oder 11, ferner umfassend Mittel, um vor der Bildung der mehrdimensionalen Matrix die Daten des optischen Signals jeder Erfassung durch Rezentrierung der zeitlichen Daten in einem Zeitbereich um eine mittlere Zeit des optischen Signals zu korrigieren.

**13.** Vorrichtung nach einem der Ansprüche 10 bis 12, umfassend Mittel zum Bestimmen von A und S durch Minimierung einer Kostenfunktion.

**14.** Vorrichtung nach einem der Ansprüche 10 bis 13, wobei die gepulste Strahlungsquelle (2, 4) ermöglicht, einen Anregungsstrahl wenigstens teilweise im Infrarot-Bereich zu erzeugen.

**15.** Vorrichtung nach einem der Ansprüche 10 bis 14, wobei die Mittel (6, 8) zum Durchführen von mindestens einer Erfassung eines optischen Signals Mittel zum Erfassen von Strahlung bei wenigstens einer Wellenlänge größer als 600 nm umfassen.

**Claims**

**1.** A method for locating at least one optical marker (22, $122_1$, $122_2$, $122_3$, $122_4$) in a diffusing medium, said marker having at least one optical property different from the diffusing medium, method wherein:

a) one performs at least one acquisition of an optical signal, characteristic for said medium (20, 120) and for one or several of said markers (22, $122_1$, $122_2$, $122_3$, $122_4$), by interaction of said medium with the radiation of a pulse radiation source (8), each acquisition including, on the one hand, one or more time components of interest, due to one or several markers, and, on the other hand, a spurious component, due to a part of said medium other than the markers,
b) a multidimensional matrix X is formed from said optical signal data of said at least one of said acquisitions,
c) said matrix X is processed by factorization into a product of only two non-negative multidimensional matrixes A and S,
d) the contribution to the optical signal of at least one of said time components is extracted from data contained in said matrixes A and S.

**2.** The method according to claim 1, further including a step of calculating the position of at least one optical marker in the diffusing medium and/or a graphical representation of the distribution of at least one such marker in said diffusing medium, from at least one of said time components.

**3.** The method according to any of claims 1 or 2, wherein said optical marker:

- comprises at least a fluorescent marker, the excitation of the medium being performed at least at one excitation wavelength of at least one fluorescent marker, the optical signal resulting from the excitation being at least at one fluorescent wavelength of at least one fluorescent marker, and wherein the fluorescence contribution due to at least one fluorescent marker is extracted from said matrices A and S;
- or comprises an area of said medium having at least one absorption and/or diffusing coefficient different from that of said surrounding diffusing medium, said optical signal resulting from the diffusion of said excitation signal into the diffusing medium (20, 120) and wherein the contribution from an area with an absorption coefficient different from that of the diffusing medium is extracted.

**4.** The method according to any of claims 1 to 3, wherein, before said multidimensional matrix is formed, the optical signal data of each acquisition are corrected by recentering the time data in a time range with respect to a pulse response time.

**5.** The method according to any of claims 1 to 4, wherein step c) comprises calculating A and S by minimizing a cost function.

6. The method according to claim 5, wherein said cost function comprises:

- the distance ∥*X-AS*∥² between the image X and the product A.S.;
- or the function:

$$F = \left\| X - AS \right\|^2 + \alpha \left\| S - S_0 \right\|^2$$

Where $S_0$ is the initial matrix and $\alpha$ is a so-called regularization coefficient.

7. The method according to any of claims 1 to 6, wherein step c) is performed by k iterations, the arrays $A_{l+1}$ and $S_{l+1}$, obtained during the 1+1-order iteration being determined from the arrays $A_l$ and $S_l$ obtained during the 1-order iteration.

8. The method according to claim 7, wherein the number of iterations is determined depending on fluctuations in the arrays A and S, or automatically depending on fluctuations in the cost function during 2 or more successive iterations.

9. The method according to any of claims 1 to 8, wherein the excitation radiation has a spectrum at least partly in infrared and/or the detected optical signal has a spectrum at least partly at a wavelength higher than 600 nm.

10. A device for identifying at least one optical marker in a diffusing medium, comprising:

a) a pulse radiation source (2, 4) for generating an excitation beam and of at least one optical marker in said medium,
b) means (6, 8) to perform at least one acquisition of an optical signal data, characteristic for said medium (20, 120) and for one or several of markers, each acquisition including, on the one hand, at least one time component of interest, due to at least one of said markers, and, on the other hand, a spurious component, due to a part of the medium other than said markers,
c) means to form a multidimensional matrix X from said optical signal data of at least one of said acquisition,
d) means (24, 27, 28) to process said matrix X into a product of only two non-negative multidimensional matrixes A and S;
e) means (24, 27, 28) to extract the contribution to the optical signal of at least one of said time components from data contained in said matrixes A and S.

11. The device according to claim 10, further comprising means to calculate the position of at least one optical marker in the diffusing medium and/or a graphical representation of the distribution of at least one marker in the diffusing medium, from at least one of said time components.

12. The device according to one of claims 10 or 11, further comprising means to correct said optical signal data of each acquisition, before said multidimensional matrix is formed, said correcting comprising recentering said time data in a time range around an average time of said optical signal.

13. The device according to one of claims 10 to 12, comprising means to determine A and S by minimizing a cost function.

14. The device according to one of claims 10 to 13, said pulsed radiation source (2, 4) producing an excitation beam at least partly in infrared.

15. The device according to one of claims 10 to 14, wherein said means (6, 8) to perform at least one acquisition of an optical signal data comprising means to detect a radiation at least at one wavelength higher than 600 nm.

FIG.1

FIG.2A

FIG.2B

source:3 det:2

FIG.3A

source:4 det:2

FIG.3B

FIG.4

Photons

Canaux temporels

X mesures

$N_X = 24$ mesures

$N_T$ canaux temporels

FIG.5

=

Matrice de poids A

$N_X$

2 columns

X

Matrice de sources S

2 lines

$N_T$

| | |
|---|---|
| Excitation du milieu Acquisition(s) | $S_1$ |
| Correction des données temporelles | $S_2$ |
| Résolution de X = A.S | $S_3$ |
| Visualisation de la position des marqueurs | $S_4$ |

FIG.6

$122_1$   $122_3$   120

$122_2$   $122_4$

FIG.7

FIG.8A

FIG.8B

FIG.9A

FIG.9B

FIG.10A

FIG.10B

FIG.11A

FIG.11B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2005065440 A
- US 20090245611 A
- JP 2008215881 A
- US 2008103390 A
- EP 10179184 A

**Littérature non-brevet citée dans la description**

- **D.WOOD et al.** Refining epifluorescence imaging and analysis with automated multiple-band flat-field correction. *Nature Methods Application Notes,* 2008
- **S. PSYCHARAKIS et al.** Autofluorescence removal from fluorescence tomography data using multispectral imaging. *Proceedings Paper SPIE,* 2007, 66260I-66260I7
- **A.T. KUMAR et al.** Feasibility of in vivo imaging of fluorescent proteins using lifetime contrast. *Optics Letters,* 2009, vol. 34, 2066-2068
- **A. LAIDEVANT et al.** Analytical method for localizing a fluorescent inclusion in a turbid medium. *Applied Optics,* 2007, vol. 46, 2131-2137
- **HERVE L et al.** Localization of fluorescence marked prostate tumor with time-resolved diffuse optical tomography. *PROCEEDINGS OF THE SPIE,* 23 Février 2010, vol. 7557
- **A, MONTCUQUET A -S et al.** Non-negative matrix factorization: a blind sources separation method to unmix fluorescence spectra. *2009 FIRST WORKSHOP ON HYPERSPECTRAL IMAGE AND SIGNAL PROCESSING: EVOLUTION IN REMOTE SENSING (WHISPERS,* 2009
- **LEE ; SEUNG.** Learning the parts of objects by non négative matrix factorization. *Nature,* 1999, 788-791
- **J.BOUTET et al.** Bimodal ultrasound and fluorescence approach for prostate cancer diagnosis. *J. Biomed. Opt.,* 2009, vol. 14, 064001
- **J.BOUTET et al.** Méthodes optiques résolues en temps pour la tomographie de fluorescence dans les milieux diffusants. *Thèse de Aurélie Laidevant,* 05 Octobre 2006